Europäisches Patentamt

European Patent Office

Office européen des brevets

(11) Publication number: **0 239 613**
**B1**

(12) **EUROPEAN PATENT SPECIFICATION**

(45) Date of publication of patent specification: **16.08.90**

(21) Application number: **86906058.2**

(22) Date of filing: **15.09.86**

(86) International application number:
**PCT/US86/01861**

(87) International publication number:
**WO 87/02066 09.04.87 Gazette 87/08**

(51) Int. Cl.⁵: **C 12 Q 1/68,** G 01 N 27/00 //
C12Q1/70

(54) **PIEZOELECTRIC DEVICE FOR DETECTION OF POLYNUCLEOTIDE HYBRIDIZATION.**

(30) Priority: **26.09.85 US 780615**

(43) Date of publication of application:
**07.10.87 Bulletin 87/41**

(45) Publication of the grant of the patent:
**16.08.90 Bulletin 90/33**

(84) Designated Contracting States:
**AT BE CH DE FR GB IT LI LU NL SE**

(56) References cited:
**EP-A- 127 438**
**WO-A-80/02201**
**WO-A-80/02518**
**WO-A-85/02627**
**US-A-4 242 096**
**US-A-4 314 821**

**Chemical Abstracts, vol. 103, no. 19, 11.11.1985, Columbus, Ohio, US; S. Wang:"Genetic substances and material sciences", p. 277, abstract no. 155873f**

(73) Proprietor: **The University of Southern Mississippi**
**Southern Station Box 10076**
**Hattiesburg Mississippi 39406-0076 (US)**

(72) Inventor: **FAWCETT, Newton, C.**
**118 Miller Street**
**Hattiesburg, MS 39401 (US)**
Inventor: **EVANS, Jeffrey, A.**
**112 Briarcliff Drive**
**Hattiesburg, MS 39401 (US)**

(74) Representative: **Szczuka, Jan Tymoteusz et al**
**Cruikshank & Fairweather 19 Royal Exchange Square**
**Glasgow G1 3AE Scotland (GB)**

## Description

The present invention generally concerns a system for detecting polynucleotide hybridization. More particularly, the invention pertains to a system for detecting polynucleotide hybridization utilizing piezoelectric crystals. The invention has particular application to medical diagnosis, bacteriology, virology, pathology, and biochemical and molecular biological research. For example, the system of the present invention is useful for detecting polynucleotide hybridizations such as DNA—RNA, DNA—DNA and RNA—RNA hybridizations.

DNA and RNA are strands of nucleic acids characterized by a phosphodiester link between the 3' position of one nucleic acid residue with the 5' position of the next nucleic acid residue. Nucleic acids contain one or more of the following bases: guanine (G), cytosine (C), adenine (A), and either thymine (T) (occurring in DNA) or uracil (U) (occurring in RNA).

In double-stranded DNA it is known that the pairing of the DNA strands are complementary, i.e., the guanine of one strand always pairs with the cytosine of the other strand (G—C), and the adenine of one strand always pairs with the thymine of the other strand (A—T). The same is true for double stranded RNA with the exception that uracil is substituted for thymine (A—U).

Pairing of single strands of polynucleotides occurs through hydrogen bonds formed between complementary bases of the nucleic acids. When heated sufficiently, the paired polynucleotides will melt; i.e., the hydrogen bonds are disrupted and the double strands are separated into individual strands (denatured). The polynucleotide strands tend to re-form duplexes once separated into single strands through the re-forming of hydrogen bonds between complementary bases.

The ability of DNA or RNA strands to reanneal with complementary strands of polynucleotides has led to the ability to determine base sequence homology through hybridization. The extent of hybridization between a DNA strand with a known base sequence, for example, and a strand of polynucleotide from a different source, can serve as a measure of species relatedness, and is useful for the purpose, among others, of identifying bacteria and viruses.

Several methods of detecting polynucleotide hybridization have been developed. One such method of detecting polynucleotide hybridization relies on radiolabelling. Other known methods make use of renaturation rates, electron microscopic determination, and the use of fluorochrome labelled polynucleotides in conjunction with fluorescence microscopy. IgG specific for RNA:DNA hybrids has also been used. Still other methods use nucleotide derivatives which contain biotin, aminobiotin, lipoic acid and other determinants attached covalently to the pyrimidine or purine ring.

The techniques employed for detecting DNA or RNA relatedness have been applied in research laboratories for several purposes, such as identifying biochemically atypical strains, classifying poorly studied groups of organisms, justifying or invalidating proposed taxonomic changes, determining the relationships between newly described organisms and existing taxons, and for identifying mixed cultures. Unfortunately, the application of these techniques in clinical studies have for most purposes been impractical. For example, in clinical studies where DNA hybridization detection employs radiolabelling, the technique has been shown to be impractical since it lacks simplicity and rapidity, is expensive, and poses potential safety hazards.

Generally, the present invention comprises a novel component and method for the detection of polynucleotide hybridization. A polynucleotide is immobilized on a surface of a piezoelectric crystal and the resonance frequency of the crystal is then noted. A variety of means for determining the resonance frequency of a piezoelectric crystal can be employed. Polynucleotide hybridization can then be detected by measuring the difference between the resonance frequency of the piezoelectric crystal before and after the prepared crystal is exposed to a separate source of polynucleotide under suitable hybridization conditions.

US Patent Specification 4242096 describes the use of a piezoelectric crystal for the determination of an antigen in a liquid sample. The crystal is coated with antigen and a predetermined amount of antibody added so as to establish competing antigen-antibody reactions. The amount of antibody bound to the antigen on the crystal is determined from the frequency change of the crystal, and this is related to the antigen concentration in the sample using a standard curve.

Specifically, the present invention provides a component for use in detecting polynucleotide hybridization comprising:

(a) a piezoelectric crystal; and
(b) a polynucleotide immobilized on a surface of said crystal.

The invention further provides a method of detecting polynucleotide hybridization between a first polynucleotide and a second polynucleotide comprising the steps of:

(a) detecting a first resonance frequency of a piezoelectric crystal wherein a first polynucleotide is attached to a surface of said crystal;

(b) exposing said attached first polynucleotide to a separate source of a second polynucleotide for a sufficient length of time and under conditions suitable for hybridization to occur between said first polynucleotide and said second polynucleotide;

(c) detecting a second resonance frequency of said crystal following said hybridization step; and

(d) comparing said first and said second resonance frequencies.

Piezoelectric detection of mass

Piezoelectricity is the electricity, or electric polarity, resulting from the application of mechanical pressure on a dielectric crystal. The concept of piezoelectricity is well known. For example, piezoelectric crystals are used in controlling frequencies in the communications field, as selective filters in electronic networks and also as sensors to accurately measure temperature.

Certain crystals exhibit a piezoelectric effect, i.e., compression of the crystals generate an electrostatic voltage across the crystals. Examples of these types of crystals include quartz, ethylene diamine tartrate, barium titanate, zincblende and the like. The frequency of a crystal depends on its effective mass. The resonant frequency of the crystal can be obtained with a frequency meter attached to the output of an oscillator circuit. If the mass of the crystal is altered, as for example, by adding material to the surface of the crystal, the resonant frequency of the crystal will change and can be measured on a frequency meter. The frequency of the crystal will lower proportionally to the amount of mass added to the crystal.

Piezoelectric crystals are commonly produced from AT or BT cut quartz plates (AT and BT refer to the orientation of the plate with respect to the crystal structure). AT and BT cut crystals vibrate in a shear mode about an axis parallel to the major surface, and have low or zero temperature coefficients at the temperature of use. The AT cut is superior to the BT cut in temperature coefficient and mass sensitivity. A typical 9 MHz crystal is a 10—16 mm disc, square or rectangle about 0.15 mm thick. A 9 MHz, AT cut quartz crystal has proven very effective in the practice of this invention.

Means for detecting the resonance frequency of a crystal can be provided in a variety of ways. Generally, a crystal will be interposed between electrode material, the leads of which are connected to an oscillator circuit. A frequency meter or the like attached to the output of the oscillator circuit is used to measure the resonance of the crystal.

The electrode material need not be in physical contact with the crystal. However, an efficient way of preparing a piezoelectric crystal for use in the present invention is to deposit electrode material on opposite surfaces of the crystal. For example, on a typical quartz crystal, the electrode material can be deposited on the central region of opposing crystal surfaces. In addition, a band of electrode material can be deposited on the crystal surface to form a pathway from the electrode material deposited on the central region of crystal surface to the edge of the crystal where leads for connecting to an oscillator circuit can be attached.

Any suitable electrode material can be used in the practice of this invention. Such materials include, but are not limited to, gold, silver, aluminum, nickel, chromium, titanium, tantalum and the like.

The instrumentation employed, such as a simple oscillator circuit, a frequency counter and a vacuum oven, and the use of micro-electric circuitry should be economically advantageous since it is inexpensive yet effective. In addition, the present invention should require only limited training to operate, especially since a signal from a piezoelectric crystal is at a frequency which can easily be displayed on a digital meter or converted to a digital signal for communication to a computer.

Bonding of polynucleotides to piezoelectric crystals

It is well known that polynucleotides will hybridize. For example, denatured DNA plus its complement will hybridize under the proper conditions and reassociate into double-stranded DNA. Hybridization of a DNA strand occurs only if incubated with its complement. Partial hybridization can occur between single strands of related species. The extent of hybridization corresponds to the extent of species relatedness. For example, hybridization of approximately 60% is considered to indicate a close relation between species.

Polynucleotides can be chemically bonded directly to a piezoelectric crystal surface or indirectly via a material previously deposited on the crystal surface. The crystal surface, for the purposes of this description, includes the crystal surface per se or any material or materials deposited on the surface of the crystal. Such other material can be electrode material or a thin layer of polymeric or other bonding material, such as intermediate molecule links. For example, a synthetic polynucleotide can be bonded chemically to the crystal surface to which a second, naturally occurring, polynucleotide can be attached by enzymatic ligation. More particularly, a polynucleotide can be ligated by enzymatic reaction (DNA or RNA ligase) to a different polynucleotide which is covalently bonded by chemical reaction to a functional group which is in turn chemically bonded to a substrate, for example, a polymeric substrate.

Particularly effective polymeric substrates are those polymers characterized by hydrolytically stable, hydrophobic backbones substituted with reactive pendant groups. For example, such polymers include, but are not limited to, copolymers of ethylene or propylene and N-(6-x-hexyl)-acrylamide, copolymers of styrene and p-x-methyl styrene, copolymers of alkylated siloxanes and 6-x-hexyl and alkyl substituted siloxanes, and similar polymers where x is a reactive group such as amino, sulfhydryl, iodo bromo, chloro, carboxyl, hydroxy, chloro carbonyl, dimethylsilyl and similar groups which are capable of combining with polynucleotides or derivatized polynucleotides. Other polymers useful in the present invention include, but are not limited to, poly-(butyl methacrylate), polyurethane and the like.

One of the most sensitive areas for immobilizing the polynucleotides is the central portion of the crystal surface.

In one embodiment of the present invention a polymer solution (usually 5 to 15 µl) is pipetted onto the crystal and the solvent is evaporated *in vacuo*. Depending upon the polymer solution used and prior experience, the crystal may be washed at this stage. The wash may consist of a combination of solvent or solvents and solutions. A typical wash would be with 100 ml of distilled water. As described above, it may be desirable to attach the polynucleotides to that portion of the crystal surface comprising the electrode material. In this instance, the polymer can be applied substantially to that area of the crystal surface.

The resonance frequency of the dry crystal can be optionally measured at this stage. If this is done, washing followed by drying *in vacuo* and measurement of the resonance frequency should be repeated until the resonance changes by less than +/− 50 Hz between successive wash-dry cycles. Prior experience may indicate the amount of washing required to achieve a stable frequency (+/− 50 Hz) at this stage. Indeed, prior experience may indicate that no wash-dry cycle is required at this point. Whether a wash cycle is needed will largely be determined by the polymer and attachment method used. The reverse side of the crystal may optionally be treated in the same way.

A buffer solution (usually phosphate buffer, pH=6 to 8) containing the polynucleotide to be immobilized is pipetted onto the crystal surface. This solution may contain additional linking reagents which react with either the polynucleotide, or with the polymer, or with both, to form chemical bonds between the polynucleotide and the polymer or other surface material. In some cases these reagents that can react with the polynucleotide, or with polymer, or with both may be applied to the crystal and dried before polynucleotide addition. Alternatively, electromagnetic radiation may be used to activate preexisting functional groups on the polymer or the polynucleotide, or both, to cause formation of chemical bonds between the polynucleotide and the substrate.

After reaction with DNA or RNA, the crystal is repeatedly washed with distilled water and dried *in vacuo* until the resonance frequency is stable within +/− 50 Hz. The temperature of the wash may be elevated, but not so high as to cause hydrolysis or other disruption of desirable bonds.

After the desired polynucleotide has been coated on the crystal surface, the crystal frequency can be recorded. This is done first by washing the crystal of all soluble and nonadhering material, and then drying the crystal in a vacuum under low heat. Afterwards, the resonance frequency of the crystal is measured and recorded.

The piezoelectric crystals, nucleic acids and other polymers used are relatively stable and inexpensive. It is contemplated that prepared piezoelectric crystals can be manufactured so inexpensively that one-time use will be feasible. Thus, one distinct advantage of the present invention is the ability to provide a variety of kits for use in the detection of polynucleotide hybridization. For example, such kits can include, as separate components, a test crystal, a control crystal, polymer, polynucleotide, linking reagent, electronic equipment, or any combination of these. In addition, the kit can include the crystal prepared to any extent desired, for example, with the polymer or other substrate or linking material only, or with the polynucleotide attached.

Piezoelectric detection of hybridization

An aqueous buffer solution containing a polynucleotide is deposited on the surface of the crystal prepared generally as described above. The polynucleotide in the solution can be either single- or double-stranded. The solution and crystal can be heated to a temperature sufficient to melt any double-stranded polynucleotides to single strands. Next, the temperature is brought to a predetermined value optimum for the hybridization of the reference polynucleotide. This incubation period is allowed to continue for a predetermined amount of time based on the polynucleotide involved. This period can last from a few minutes to several hours depending on the complexity of the polynucleotide.

In one embodiment, hybridization is carried out by incubating on the surface of a crystal a buffered solution of polynucleotide. Typically the solution will contain about 1 milligram/milliliter of the polynucleotide to be tested, but can have a substantially lower concentration. A typical incubation temperature would be approximately 30°C below the melting temperature of the duplex to be formed. The incubation time will vary from a few minutes to several hours depending upon the complexity and molecular weight of the polynucleotide to be duplexed. A useful temperature regimen involves heating the polynucleotide solution above its melting point and applying the hot solution to the crystal. Then the temperature is lowered to the incubation temperature and held there for the required time. Alternatively, a melted solution of DNA or RNA can be rapidly cooled, i.e., quick cooled, to slow the spontaneous association of complementary DNA or RNA strands.

The quick cooled solution is then applied to the crystal and incubated at the optimum temperature for duplex formation. In many cases it is possible to use solvent systems which lower the association temperature. For example, use of 50% formamide allows effective incubation at lower temperatures in cases where an elevated temperature would be required without formamide.

Typically 1 to 50 microliters of polynucleotide solution is applied to one or both sides of the crystal. The solution should be retained on the crystal during incubation without significant evaporation. This can be done, for example, by forming a silicone rubber reaction chamber (dome) over the crystal's central region. The reaction chamber can be made from a silicone gas chromatograph septum with the center portion removed and covered with a second intact septum. Alternatively, a reaction chamber specially manufactured to accommodate piezoelectric crystals can be used.

Following the incubation period, the crystal is washed free of soluble and nonadhering material and dried under a vacuum. If the applied solution contained a polynucleotide which is the complement of the reference polynucleotide, then the mass of polynucleotide on the crystal surface should be increased. Generally, the reference polynucleotide will be immobilized on the crystal surface prior to incubation with the test polynucleotide. However, it is posible to reverse this order, i.e., immobilize the test polynucleotide on the crystal surface and then incubate the crystal with the reference polynucleotide.

This system is capable of providing both qualitative and quantitative detection of hybridization. The amount of the increase in mass caused by hybridization of the polynucleotides is generally measured with a frequency meter. The extent of hybridization is evaluated from the difference in resonance frequency before and after the incubation step. For example, based on a 9 MHz crystal, an approximately 0.8 hertz shift corresponds to a one nanogram change in mass. The sensitivity of the detection is a function of the fundamental frequency of the crystal. A higher fundamental frequency of the crystal corresponds to an increase in sensitivity, while a lower fundamental frequency will give the opposite result.

In addition, it is contemplated that this system will provide numerous advantages over other known techniques, especially in clinical work where rapid and accurate, yet economically obtained, results are required. The subject invention provides faster results due to the basic simplicity of piezoelectric mass detection and can be applied to mixed bacterial or viral cultures whereas non-hybridization methods of identifying bacteria and viruses generally require that a pure culture (non-mixed) be obtained. In addition, the present invention should provide an advantage over older methods of pathogen identification. The older methods are based on the observation of phenotypes which can fail to correctly identify mutated species which can otherwise be detected by hybridization techniques. Moreover, mutagenic dyes, as used in fluorescence assay methods, and [32]p or equally hazardous isotopes, as used in radio assay methods, are avoided.

Other diagnostic uses are also contemplated as being within the scope of the present invention. For example, it is contemplated that the present invention will be useful for diagnosing the presence of nucleic acid-containing etiological agents, testing bacterium to determine the presence of resistance to an antibiotic, detecting genetic disorders, diagnosing tumor cells and other similar techniques where polynucleotide hybridization is employed.

The following examples are given to more fully illustrate the present invention, and shall not be construed as limiting the scope of the invention in any way. These examples are representative of the immobilization of polynucleotides on crystals, their subsequent hybridization and the detection of hybridization by their piezoelectric resonance. Therefore, the method is not limited to only those DNAs or RNAs enumerated.

Examples

The procedures used in the following Examples follow the general procedures discussed above. The procedures were carried out in parallel on two separate crystals, one of which was designated as a control. The frequency shifts obtained upon hybridization are expressed relative to the control.

The control was processed in the same manner as the test sample except that the conditions for the control were adjusted so that significant hybridization would not occur.

The results are expressed as follows:

$$\Delta Hz = (Hz_2 - Hz_3)_{control} - (Hz_2 - Hz_3)_{test} \text{ sample}$$

where:

$Hz_2$ = the resonance frequency of the prepared crystal, and

$Hz_3$ = the resonance frequency of the crystal following the incubation period.

Hybridization is generally indicated when $\Delta H$ has a value from minus a few hundred to minus several thousand Hertz. The reproducibility of $\Delta Hz$ depends on various factors such as the reproducibility of the polynucleotide immobilization and the subsequent hybridization conditions and reactions.

RNA Hybridization detected by piezoelectric resonance

It is well known that hybridization will occur between polyinosinic acid (poly I) and polycytidylic acid (poly C) to form a double-stranded synthetic polymer (poly I:C). However, hybridization will not occur between polyuridylic acid (poly U) and polyinosinic acid (poly I).

Examples 1—3 below demonstrate the ability to measure specific hybridization which can occur on the crystal surface.

Example 1

Polycytidylic acid was hybridized with Polyinosinic acid attached to Poly(butyl methacrylate) substrate as follows:

10 microliters of poly(butyl methacrylate) (0.1% in ethyl acetate) was applied to both sides of the crystal and dried. 10 microliters of triazido trinitro benzene linker (0.1% in chloroform) was applied to both sides of the crystal. 5 microliters of 1 milligram/milliliter poly I in buffer A (0.14 Molar sodium phosphate, pH 6.8) was applied to both sides of the polymer coated crystal and irradiated at 300 nanometers for 30 minutes on each side. 5 microliters of 1 milligram/milliliter poly C in buffer A was applied for hybridization. Hybridization was done at 46°C for 25 minutes under a glass dome.

The control crystal was carried through the identical procedure without polyinosinic acid

First Result:
ΔHz=−613 versus control
Second Result:
ΔHz=−686 versus control.

## Example 2

Polycytidylic acid was hybridized with Polyinosinic acid attached to a polyurethane substrate as follows:

5 microliters of polyurethane (0.2% in dimethylformamide) was applied to both sides of the crystal and dried. 5 microliters of 2,6-bis(4-azidobenzylidene)-cyclohexanone linker (0.1% in acetone) was applied to both sides of the crystal. 5 microliters of 5 milligram/milliliter poly I in buffer B (0.10 Molar sodium, 0.01 Molar phosphate, pH 7.8) was applied to both sides of the polymer coated crystal and irradiated at 330 nanometers for 30 minutes on each side. 5 microliters of $2.0\times10^{-3}$ Molar poly C (0.65 milligram/milliliter) in buffer B was applied to both sides of the crystal for hybridization. The crystal was heated with poly C to 65°C for 30 minutes and the hybridization was carried out at room temperature for 2 hours with silicone rubber domes.

The control crystal was carried through an identical procedure without polyinosinic acid.

Result:
ΔHz=−1237 versus control.

## Example 3

Polycytidylic acid was hybridized with polyinosinic acid attached to a polyurethane substrate with cyclohexene reactive site mask as follows:

10 microliters of polyurethane (0.2% in dimethylformamide) were added to both sides of the crystal and dried. 10 microliters of 2,6-bis(4-azidobenzylidene)-cyclohexanone linker (0.2% in acetone) was then added to both sides of the crystal. 5 microliters of $2.0\times10^{-3}$ Molar poly I (0.70 milligram/milliliter) in buffer B (0.10 Molar sodium, 0.10 Molar phosphate, pH 7.8) was applied to both sides of the polymer coated crystal and irradiated at 366 nanometers for 0.5 hours. 10 microliters of cyclohexene was applied to each side of the crystal and irradiated at 366 nanometers for 10 minutes on each side. 5 microliters of $2.0\times10^{-3}$ Molar poly C (0.65 milligram/milliliter) in buffer B was applied to both sides of the crystal for hybridization. The crystal was heated to 60°C with the poly C for 30 minutes and then incubated for 2 hours at room temperature for hybridization.

The control crystal was carried through the identical procedure, but using 5 microliters of $2.0\times10^{-3}$ Molar polyuridylic acid (0.65 milligram/milliliter) in place of polycytidylic acid.

Result:
ΔHz=−227 Hz versus control.

The control (Hz$_3$−Hz$_2$) was +18 Hz demonstrating that there was not apparent hybridization between the polyinosinic acid immobilized on the crystal and polyuridylic acid present in solution. This is consistent with the known specificity of polyinosinic acid for hybridization to poly C but not to poly U.

DNA Hybridization detected by piezoelectric resonance

## Example 4

*Salmonella typhimurium* (*S. typhimurium*) DNA was hybridized to *S. typhimurium* DNA attached to a poly(butyl methacrylate) substrate as follows:

10 microliters of a 100:1 (volume/volume) solution of poly(butyl methacrylate) (0.1% in ethyl acetate) and dimethoxyphenylacetophenone (0.1% in acetone) was applied to the crystal. 10 microliters of 1 milligram/milliliter *S. typhimurium* DNA (denatured by boiling and then quick cooling at 4°C) in buffer A (0.14 Molar sodium phosphate, pH 6.8) was applied to one side of the crystal and irradiated at 365 nanometers for 2 hours. The crystal was rinsed with distilled water and dried. In the case of Result 1 below, an additional 10 microliters of denatured *S. typhimurium* DNA was applied and irradiated as above. Hybridization was carried out with 10 microliters of 1 milligram/milliliter melted *S. typhimurium* DNA (heated at 100°C and then cooled to 65°C). Incubation was at 65°C for 2 hours with a silicone rubber dome.

The control crystal was carried through the identical procedure but incubated with 20 microliters of 0.4 milligram/milliliter of melted calf thymus DNA instead of melted *S. typhimurium* DNA during the hybridization step.

First Result:
ΔHz=−779 versus control
Second Result:
ΔHz=−1013 versus control.

## Example 5

*S. typhimurium* DNA was hybridized to *S. typhimurium* DNA attached to a poly(butyl methacrylate) substrate as follows:

10 microliters of a 100:1 (volume/volume) solution of poly(butyl methacrylate) (0.1% in ethyl acetate) and dimethoxyphenylacetophenone (0.1% in acetone) was applied to the crystal. The crystal was then dipped into the poly(butyl methacrylate):dimethoxyphenylacetophenone solution having the above composition. 10 microliters of 1 milligram/milliliter *S. typhimurium* (denatured by boiling and then quick cooling at 4°C) in buffer A (0.14 Molar sodium phosphate, pH 6.8) was applied to one side of the crystal and irradiated at 365 nanometers for 2 hours. Hybridization was carried out with 10 microliters of 1 milligram/milliliter melted *S. typhimurium* DNA (heated at 100°C and then cooled to 65°C) and incubated for 2 hours with a silicone rubber dome.

The control crystal was carried through the identical procedure, but denatured calf thymus DNA (25 microliters of 0.4 milligram/milliliter in buffer A) was used for immobilization instead of denatured *S. typhimurium* DNA.

Result:
ΔHz=−833 versus control.

Example 6

*S. typhimurium* DNA was hybridized to endonuclease restricted *S. typhimurium* DNA inserted into single-stranded M13 viral DNA attached to poly(butyl methacrylate) as follows:

5 microliters of a 100:1 (volume/volume) solution of poly(butyl methacrylate) (0.1% in ethyl acetate) and dimethoxyphenylacetophenone (0.1% in acetone) was applied to the crystal. 10 microliters of 1 milligram/milliliter single-stranded M13 mp8 with *Salmonella* DNA insert in buffer B (0.10 Molar sodium, 0.10 Molar phosphate, pH 7.8) was applied to a polymer coated crystal and irradiated at 365 nanometers for 2 hours. 10 microliters of 1 milligram/milliliter blender sheared (4 minutes), melted *S. typhimurium* DNA was applied for hybridization at 65°C for 2 hours using silicone rubber domes.

Control 1: The crystal was carried through an identical procedure but using 10 microliters of 1 milligram/milliliter of blender sheared, melted calf thymus DNA for the hybridization step instead of *S. typhimurium* DNA.

Control 2: The crystal was carried through an identical procedure, but using buffer B instead of M13 mp8 DNA with *Salmonella* DNA insert, and using 10 microliters of 1 milligram/milliliter blender sheared, melted calf thymus DNA for the hybridization step instead of *S. typhimurium* DNA.

Result:
ΔHz=−557 versus control 1
ΔHz=−875 versus control 2.

**Claims**

1. A component for use in detecting polynucleotide hybridization comprising:
   (a) a piezoelectric crystal; and
   (b) a polynucleotide immobilized on a surface of said crystal.

2. The component of claim 1 further comprising means for determining the resonance frequency of said crystal.

3. The component of claim 2 wherein said means for determining the resonance frequency of said crystal includes electrode material deposited on at least a portion of opposite surfaces of said crystal.

4. The component of claim 3 wherein a substantial portion of said polynucleotide is immobilized on that portion of said crystal surface comprising said electrode material.

5. The component of claim 1 or 4 wherein said crystal surface to which said polynucleotide is immobilized further comprises a polymeric substrate intermediate between the crystal and polynucleotide layer.

6. The component of claim 1 or 4 wherein said polynucleotide is immobilized on a surface of said crystal by incorporating a linker between said polynucleotide and said surface to which said polynucleotide is to be immobilized.

7. The component of claim 1 or 4 wherein said polynucleotide is immobilized on a surface of said crystal by enzymatically ligating said polynucleotide using DNA or RNA ligase to a different polynucleotide in order to then covalently bond said different polynucleotide by chemical reaction to a functional group which is in turn chemically bonded to said crystal surface.

8. The component of claim 1 wherein said polynucleotide is DNA or RNA.

9. The component of claim 3 wherein said electrode material comprises a metal selected from gold, silver, aluminum, nickel, chromium, titanium, tantalum.

10. The component of claim 5 wherein said polymeric substrate is selected from a class of polymers characterized by hydrolytically stable, hydrophobic backbones substituted with reactive pendant groups.

11. The component of claim 10 wherein said polymer is selected from copolymers of styrene and p-x-methylstyrene, ethylene or propylene and N-(6-x-hexyl)-acrylamide, alkylated siloxanes and 6-x-hexyl and alkyl substituted siloxanes, where X is a reactive group capable of combining with polynucleotides or derivatized polynucleotides.

12. The component of claim 5 wherein said polymeric substrate comprises a synthetic polynucleotide.

13. The component of claim 5 wherein said polymeric substrate comprises a polymer selected from poly(butyl methacrylate), polyurethane.

14. The component of claim 6 wherein said linker is an organic compound characterized by the presence of an azido group.

15. The component of claim 14 wherein said linker is selected from 2,6-bis(4-azidobenzylidene)-cyclohexanone, triazido trinitro benzene.

16. A device for detecting the relatedness between a given polynucleotide and another polynucleotide, comprising a piezoelectric crystal, a surface of said crystal having said given polynucleotide attached thereto, said crystal being in electrical cooperation with a means for determining the resonance frequency of said crystal.

17. A device for detecting polynucleotide hybridization comprising:
    (a) a piezoelectric crystal;
    (b) a polynucleotide attached to a surface of said crystal; and
    (c) a means for determining the resonance frequency of said crystal.

18. The device of claim 17 wherein said surface to which a substantial portion of said polynucleotide is attached comprises an electrode material deposited on at least a portion of said crystal.

19. The device of claim 17 wherein said surface to which a substantial portion of said polynucleotide is attached comprises a polymer coated on at least a portion of said crystal.

20. The device of claim 17 wherein said crystal is a quartz crystal.

21. The device of claim 18 wherein said electrode material is a metal selected from gold, silver, chromium, nickel, aluminum, titanium, tantalum.

22. The device of claim 17 wherein said polynucleotide is attached to said surface by a linker.

23. The device of claim 19 wherein said polymer is selected from a class of polymers characterized by hydrolytically stable, hydrophobic backbones substituted with reactive pendant groups.

24. The device of claim 23 wherein said polymer is selected from copolymers of styrene and p-x-methylstyrene, ethylene or propylene and N-(6-x-hexyl)-acrylamide, alkylated siloxanes and 6-x-hexyl and alkyl substituted siloxanes, where X is a reactive group capable of combining with polynucleotides or derivatized polynucleotides.

25. The device of claim 19 wherein said polymer is selected from the group comprising poly(butyl methacrylate), polyurethane.

26. The device of claim 19 wherein said polymer is a synthetic polynucleotide.

27. The device of claim 22 wherein said linker is selected from the group comprising organic compounds characterized by the presence of an azido group.

28. A method of detecting polynucleotide hybridization between a first polynucleotide and a second polynucleotide comprising the steps of:

(a) detecting a first resonance frequency of a piezoelectric crystal wherein a first polynucleotide is attached to a surface of said crystal;

(b) exposing said attached first polynucleotide to a separate source of a second polynucleotide for a sufficient length of time and under conditions suitable for hybridization to occur between said first polynucleotide and said second polynucleotide;

(c) detecting a second resonance frequency of said crystal following said hybridization step; and

(d) comparing said first and said second resonance frequencies.

**Patentansprüche**

1. Komponente zur Verwendung beim Nachweis von Polynukleotid-Hybridisierung, welche

(a) einen piezoelektrischen Kristall und

(b) ein auf der Oberfläche dieses Kristalls immobilisiertes Polynukleotid aufweist.

2. Komponente nach Anspruch 1, die außerdem Mittel zum Nachweis der Resonanzfrequenz dieses Kristalls aufweist.

3. Komponente nach Anspruch 2, in der diese Vorrichtung zum Nachweis der Resonanzfrequenz dieses Kristalls Elektrodenmaterial enthält, das mindestens auf einen Teil der gegenüberliegenden Obeflächen dieses Kristalls aufgetragen wird.

4. Komponente nach Anspruch 3, in der ein wesentlicher Teil dieses Polynukleotids auf diesem Teil dieser Kristalloberfläche, die dieses Elektrodenmaterial aufweist, immobilisiert ist.

5. Komponente nach Anspruch 1 oder 4, in der diese Kristalloberfläche, auf welcher dieses Polynukleotid immobilisiert ist, zusätzlich ein polymeres Zwischensubstrat zwischem dem Kristall und der Polynukleotidschicht aufweist.

6. Komponente nach Anspruch 1 oder 4, in der dieses Polynukleotid auf einer Oberfläche dieses Kristalls immobilisiert ist, in dem ein Bindeglied zwischen dieses Polynukleotid und diese Oberfläche, an welcher dieses Polynukleotid immobilisiert wird, eingebaut wird.

7. Komponente nach Anspruch 1 oder 4, in der dieses Polynukleotid auf einer Oberfläche dieses Kristalls immobilisiert wird, in dem dieses Polynukleotid unter Verwendung von DNA oder RNA-Ligase enzymatisch an ein anderes Polynukleotid gebunden wird, um dieses andere Polynukleotid dann kovalent mittels chemischer Reaktion an eine funktionelle Gruppe zu binden, welche ihrerseits chemisch an diese Kristalloberfläche gebunden ist.

8. Komponente nach Anspruch 1, in der dieses Polynukleotid DNA oder RNA ist.

9. Komponente nach Anspruch 3, in der dieses Elektrodenmaterial ein Metall enthält, das aus Gold, Silber, Aluminium, Nickel, Chrom, Titan oder Tantal ausgewählt ist.

10. Komponente nach Anspruch 5, in der dieses polymere Substrat aus einer Klasse von Polymeren ausgewählt ist, welche durch hydrolytisch stabile, hydrophobe Grundgerüste charakterisiert sind, die mit reaktiven Seitengruppen substituiert sind.

11. Komponente nach Anspruch 10, worin dieses Polymer ausgewählt ist aus Copolymeren von Styrol und p-x-Methylstyrol, Ethylen oder Propylen und N-(6-x-Hexyl)-acrylamid, alkylierten Siloxanen und 6-x-Hexyl und alkylsubstituierten Siloxanen, worin X ein reaktive Gruppe ist, die zur Verbindung mit Polynukleotid oder derivatisierten Polynukleotiden befähigt ist.

12. Komponente nach Anspruch 5, in der dieses polymere Substrat ein synthetisches Polynukleotid enthält.

13. Verbindung nach Anspruch 5, in der dieses polymere Substrat ein Polymer enthält, das aus Poly-(butylmethacrylat), Polyurethan ausgewählt ist.

14. Komponente nach Anspruch 6, in der dieses Verbindungsglied eine durch die Gegenwart einer Azidogruppe charakterisierte organische Verbindung ist.

15. Verbindung nach Anspruch 14, in der dieses Bindeglied aus 2,6-bis(4-Azidobenzyliden)-cyclohexanon, Triazido-trinitrobenzol ausgewählt ist.

16. Vorrichtung zum Nachweis der Verwandschaft zwischen einem vorgegebenen Polynukleotid und einem anderen Polynukleotid, die einen piezoelektrischen Kristall aufweist, wobei eine Oberfläche dieses Kristalls dieses vorgegebene Polynukleotid daran befestigt hat, und dieser Kristall elektrisch mit einer Vorrichtung zur Bestimmung der Resonanzfrequenz dieses Kristalls zusammenarbeitet.

17. Vorrichtung zum Nachweis von Polynukleotid Hybridisierung, die

(a) einen piezoelektrischen Kristall,

(b) ein an der Oberfläche diese Kristalls befestigtes Polynukleotid und

(c) eine Vorrichtung zur Bestimmung der Resonanzfrequenz dieses Kristalls aufweist.

18. Vorrichtung nach Anspruch 17, in der diese Oberfläche, an der ein wesentlicher Teil dieses Polynukleotids befestigt ist, ein auf mindestens einem Teil dieses Kristalls abgeschiedenes Elektrodenmaterial umfaßt.

19. Vorrichtung nach Anspruch 17, in dem diese Oberfläche, an die ein wesentlicher Teil dieses Polynukleotids befestigt ist, ein auf mindestens einen Teil dieses Kristalls beschichtetes Polymer aufweist.

20. Vorrichtung nach Anspruch 17, in dem dieser Kristall ein Quarzkristall ist.

21. Vorrichtung nach Anspruch 18, in dem dieses Elektrodenmaterial ein aus Gold, Silber, Chrom, Nickel, Aluminium, Titan und Tantal ausgewähltes Metall ist.

22. Vorrichtung nach Anspruch 17, in dem dieses Polynukleotid an diese Oberfläche mittels eines Bindesglieds befestigt ist.

23. Vorrichtung nach Anspruch 19, in dem dieses Polymer aus einer Klasse von Polymeren ausgewählt ist, die durch hydrolytisch stabile, hydrophobe Grundgerüste charakterisiert sind, die mit reaktiven Seitengruppen substituiert sind.

24. Vorrichtung nach Anspruch 23, in dem dieses Polymer aus Copolymeren von Styrol und p-x-Methylstyrol, Ethylen oder Propylen und N-(6-x-Hexyl)-Acrylamid, alkylierten Siloxanen und 6-x-Hexyl- und Alkyl- substituierten Siloxanen ausgewählt ist, in denen X eine reaktive Gruppe ist, die zur Verbindung mit Polynukleotiden oder derivatisierten Polynukleotiden befähigt ist.

25. Vorrichtung nach Anspruch 19, in dem dieses Polymer aus der Gruppe ausgewählt ist, die Poly-(butylmethacrylate) und Polyurethan aufweist.

26. Vorrichtung nach Anspruch 19, in der dieses Polymer ein synthetisches Polynukleotid ist.

27. Vorrichtung nach Anspruch 22, in der dieses Bindeglied aus der Gruppe ausgewählt ist, die organische Verbindungen aufweist, welche durch die Gegenwart einer Azidogruppe charakterisiert sind.

28. Verfahren zum Nachweis von Polynukleotid-Hybridisierung zwischen einem ersten Polynukleotid und einem zweiten Polynukleotid, welches die folgenden Schritte umfaßt:

(a) Nachweis einer ersten Resonanzfrequenz eines piezoelektrischen Kristalls, in dem ein erstes Polynukleotid an eine Oberfläche dieses Kristalls angeheftet ist,

(b) Aussetzen diese ersten angehefteten Polynukleotids einer getrennten Quelle eines zweiten Polynukleotids für eine genügend lange Zeit und unter Bedingungen, die zur Hybridisierung geeignet sind, welche zwischen diesem ersten Polynukleotid und diesem zweiten Polynukleotid auftritt,

(c) Nachweis einer zweiten Resonanzfrequenz dieses Kristalls, die auf diesen Hybridesierungsschritt folgt, und

(d) Vergleich dieser ersten und dieser zweiten Resonanzfrequenzen.

## Revendications

1. Composant utile dans la détection de l'hybridation de polynucléotides, caractérisé en ce qu'il comprend:

(a) un cristal piézoélectrique; et

(b) un polynucléotide immobilisé sur une surface de ce cristal.

2. Composant suivant la revendication 1, caractérisé en ce qu'il comprend de plus des moyens pour déterminer la fréquence de résonance de ce cristal.

3. Composant suivant la revendication 2, caractérisé en ce que ces moyens pour déterminer la fréquence de résonance de ce cristal comprennent un matériau d'électrode déposé sur au moins une partie des surfaces opposées de ce cristal.

4. Composant suivant la revendication 3, caractérisé en ce qu'une notable portion de ce polynucléotide est immobilisée sur la partie de la surface de ce cristal comprenant ce matériau d'électrode.

5. Composant suivant la revendication 1 ou 4, caractérisé en ce que la surface du cristal sur laquelle est immobilisé ce polynucléotide, comprend de plus un substrat polymère intermédiaire entre le cristal et la couche de polynucléotide.

6. Composant suivant la revendication 1 ou 4, caractérisé en ce que ce polynucléotide est immobilisé sur une surface de ce cristal par incorporation d'un linker entre ce polynucléotide et la surface sur laquelle est immobilisé ce polynucléotide.

7. Composant suivant la revendication 1 ou 4, caractérisé en ce que ce polynucléotide est immobilisé sur une surface de ce cristal par ligature enzymatique de ce polynucléotide au moyen d'une ADN ou ARN ligase à un polynucléotide différent, afin de lier ensuite par covalence ce polynucléotide différent par réaction chimique à un groupe fonctionnel lequel est à son tour lié chimiquement à cette surface du cristal.

8. Composant suivant la revendication 1, caractérisé en ce que ce polynucléotide est un ADN ou un ARN.

9. Composant suivant la revendication 3, caractérisé en ce que ce matériau d'électrode comprend un métal choisi parmi l'or, l'argent, l'aluminium, le nickel, le chrome, le titane et le tantale.

10. Composant suivant la revendication 5, caractérisé en ce que ce substrat polymère est choisi dans une classe de polymères caractérisés par des squelettes hydrophobes, stables hydroly- tiquement substitués avec des groupes réactifs pendants.

11. Composant suivant la revendication 10, caractérisé en ce que ce polymère est choisi parmi des copolymères de styrène et de p-x-méthylstyrène, d'éthylène ou de propylène et de N-(6-x-hexyl)-acrylamide, de siloxanes alkylés et de siloxanes substitués par des groupes 6-x-hexyle et alkyle, où x est un groupe réactif capable de se combiner avec des polynucléotides ou des dérivés de polynucléotides.

12. Composant suivant la revendication 5, caractérisé en ce que ce substrat polymère comprend un polynucléotide synthétique.

13. Composant suivant la revendication 5, caractérisé en ce que ce substrat polymère comprend un polymère choisi parmi un poly-(méthacrylate de butyle) et un polyuréthanne.

14. Composant suivant la revendication 6, caractérisé en ce que ce linker est un composé organique caractérisé par la présence d'un groupe azido.

15. Composant suivant la revendication 14, caractérisé en ce que ce linker est choisi parmi la 2,6-bis(4-azidobenzylidène)-cyclohexanone et le triazido trinitro benzène.

16. Dispositif pour détecter la parenté entre un polynucléotide donné et un autre polynucléotide, caractérisé en ce qu'il comprend un cristal piézoélectrique sur une surface duquel est attaché ce polynucléotide donné, ce cristal étant en coopération électrique avec des moyens pour déterminer la fréquence de résonance de ce cristal.

17. Dispositif pour détecter une hybridation de polynucléotides, caractérisé en ce qu'il comprend:

(a) un cristal piézoélectrique;

(b) un polynucléotide fixé sur une surface de ce cristal; et

(c) des moyens pour déterminer la fréquence de résonance de ce cristal.

18. Dispositif suivant la revendication 17, caractérisé en ce que cette surface sur laquelle est attachée une notable portion de ce polynucléotide comprend un matériau d'électrode déposé sur au moins une partie de ce cristal.

19. Dispositif suivant la revendication 17, caractérisé en ce que cette surface sur laquelle est attachée une notable portion de ce polynucléotide comprend un polymère déposé sur au moins une partie de ce cristal.

20. Dispositif suivant la revendication 17, caractérisé en ce que ce cristal est un cristal de quartz.

21. Dispositif suivant la revendication 18, caractérisé en ce que ce matériau d'électrode est un métal choisi parmi l'or, l'argent, l'aluminium, le nickel, le chrome, le titane et le tantale.

22. Dispositif suivant la revendication 17, caractérisé en ce que ce polynucléotide est fixé à cette surface au moyen d'un linker.

23. Dispositif suivant la revendication 19, caractérisé en ce que ce polymère est choisi dans une classe de polymères caractérisés par des squelettes hydrophobes, stables hydrolytiquement substitués avec des groupes réactifs pendants.

24. Dispositif suivant la revendication 23, caractérisé en ce que ce polymère est choisi parmi des copolymères de styrène et de p-x-méthylstyrène, d'éthylène ou de propylène et de N-(6-x-hexyl)-acrylamide, de siloxanes alkylés et de siloxanes substitués par des groupes 6-x-hexyle et alkyle, où x est un groupe réactif capable de se combiner avec des polynucléotides ou des dérivés de polynucléotides.

25. Composant suivant la revendication 19, caractérisé en ce que ce polymère est choisi parmi un poly(méthacrylate de butyle) et un polyuréthanne.

26. Dispositif suivant la revendication 19, caractérisé en ce que ce polymère est un polynucléotide synthétique.

27. Dispositif suivant la revendication 22, caractérisé en ce que ce linker est choisi dans le groupe comprenant des composés organiques caractérisés par la présence d'un groupe azido.

28. Procédé pour détecter une hybridation entre un premier polynucléotides et un second polynucléotide, caractérisé en ce qu'il comprend les étapes de:

(a) détection d'une première fréquence de résonance d'un cristal piézoélectrique sur une surface duquel est attaché un premier polynucléotide;

(b) exposition de ce premier polynucléotide attaché à une source distincte d'un second polynucléotide pendant une période de temps suffisante et dans des conditions convenables pour qu'apparaisse une hybridation entre ce premier polynucléotide et ce second polynucléotide;

(c) détection d'une seconde fréquence de résonance de ce cristal à la suite de cette étape d'hybridation; et

(d) comparaison de ces première et seconde fréquences de résonance.